# EUROPEAN PATENT SPECIFICATION

(11) **EP 2 614 057 B1**
(45) Date of publication and mention of the grant of the patent: **21.10.2015**
(21) Application number: 11757246.1
(22) Date of filing: 06.09.2011
(51) Int. Cl.: C07D 319/06, C07D 319/08, C07C 229/08

(54) **SALTS OF 7-AMINO-3,5-DIHYDROXYHEPTANOIC ACID ESTERS**
SALZE AUS 7-AMINO-3,5-DIHYDROXYHEPTANONSÄUREESTERN
SELS D'ESTERS D'ACIDES 7-AMINO-3,5-DIHYDROXYHEPTANOÏQUES

(30) Priority: 11.01.2011 EP 11150571; 09.09.2010 EP 10175968
(43) Date of publication of application: 17.07.2013
(73) Proprietor: DSM Sinochem Pharmaceuticals Netherlands B.V., 2613 AX Delft (NL)
(72) Inventor: LANGE, DE, Ben, NL-6151 GE Munstergeleen (NL); ELSENBERG, Henricus, Leonardus, Marie, NL-5935 CD Steyl (NL)
(74) Representative: De Vroom, Erik
(86) International application number: PCT/EP2011/065375
(87) International publication number: WO 2012/032035

(56) References cited:
- WO-A1-2004/096788
- WO-A2-89/07598
- US-A- 5 103 024
- US-A- 5 155 251
- HONG WOO LEE ET AL.: "An efficient method for the large-scale synthesis of atorvastatin calcium", BIOMOLECULES & THERAPEUTICS, vol. 16, no. 1, 2008, pages 28-33, XP002614444,

## Description

### Field of the invention

The present invention relates to novel salts of (3*R*,5*R*)-7-amino-3,5-dihydroxyheptanoic acid esters and a process for the preparation thereof.

### Background of the invention

The *tert*-butyl ester of (3*R*,5*R*)-7-amino-3,5-dihydroxyheptanoic acid (**1**, R₁ = R₂ = H, R₃ = -C(CH₃)₃) 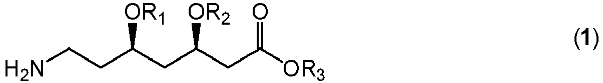 is a compound known (see *e.g.* US 5,103,024, US 5,155,251 and WO 2000/68221) for its use as intermediate in the synthesis of atorvastatin. Prior to further conversion into atorvastatin the hydroxyl groups of the heptanoic acid derivative mentioned above are protected. Atorvastatin ([*R*-(*R**,*R**)]-2-(4-fluorophenyl)-β,δ-dihydroxy-5-(1-methylethyl)-3-phenyl-4-[(phenylamino)carbonyl]-1*H*-pyrrole-1-heptanoic acid hemi calcium salt) is a pharmaceutical ingredient useful as an inhibitor of the enzyme 3-hydroxy-3-methylglutaryl-coenzyme A reductase (HMG-CoA reductase) and thus useful as a hypolipidemic and hypocholesterolemic agent.

The prior art processes for the preparation of the *tert*-butyl ester of (**1**) mentioned above such as outlined in US 5,103,024 and US 5,155,251 have several disadvantages such as the need of complicated purification techniques like fractional distillation or column chromatography. In WO 2000/68221 a crystallization technique was disclosed based on the formation of salts. According to the latter document the *tert*-butyl ester of (3*R*,5*R*)-7-amino-3,5-dihydroxyheptanoic acid (**1**, R₁,R₂ forming a cyclic acetal with -C(CH₃)₃, R₃ = -C(CH₃)₃) forms with organic acids, notably pivalic acid, salts that can be crystallized prior to further conversion into atorvastatin. Unfortunately formation of salts and/or crystals are highly unpredictable processes even for compounds that are structurally closely related. Particularly *tert*-butyl esters are known to the skilled person to be compounds that display crystallization characteristics that are quite extraordinary when compared to many other aspects, probably as a result of the bulkiness of *tert*-butyl esters. Many compounds have been obtained in solid crystalline form only after lengthy laboratory procedures that often involve one or more inventive manipulations such as the use of non-obvious solvents or co-solvents, physical manipulations such as contacting with foreign objects, irradation, ultrasound and many more. Not seldomly only an unpredictable combination of the above manipulations leads to the desired result, if at all. Hence defining alternate and improved carboxylic acid protection for compounds of general formula (**1**) may be a first general need in atorvastatin production, finding conditions that would result in isolation of such an intermediate in a highly pure crystalline form is, given the unpredictability of crystallization, a still larger challenge.

(Further reference is made to WO2004/096788, WO89/07598 and to Hong Woo Lee et al., "Biomolecules & Therapeutics", 16(1), 2008, pages 2833).

### Detailed description

In a first aspect, the present invention relates to novel salts of acids with 2-propyl esters of general formula (**2**) 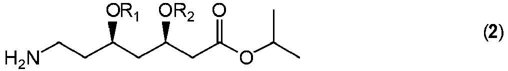 wherein R₁ and R₂ are independently chosen from the list consisting of ethyl, hydrogen, methyl and propyl or combined into a diol protecting group such that the compound of general formula (**2**) is represented as a compound of formula (**2a**), (**2b**) or (**2c**)

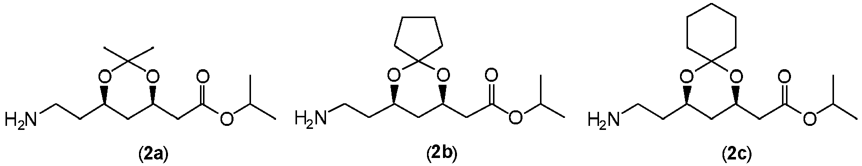

The 2-propyl esters of general formula (**2a**), (**2b**) and (**2c**) are mentioned in WO 2004/096788 and WO 89/07598 and have advantages over the well-known *tert*-butyl esters as the latter require cumbersome introduction by means of isobutene. Moreover the 2-propyl esters have a lower molecular weight leading to a lower environmental burden and are easier to re-use as the liberated 2-propyl alcohol can be re-used as such whereas in case of *tert*-butyl esters the liberated *tert*-butanol first needs to undergo chemical manipulation before re-use as a protecting group is possible.

A major advantage of crystalline intermediates is that their isolation offers the opportunity for introducing a purification step in the production process. Unfortunately however, hitherto there was no report of stable salts of esters other than the well-studied *tert*-butyl esters. Thus the 2-propyl esters of general formula (**2**) remained relatively unattractive for the preparation of atorvastatin as no straightforward and simple purification methodology was available. As 2-propyl esters are known to be more sensitive towards hydrolysis (compared to *tert*-butyl esters), the more surprising it is that stable salts of 2-propylesters can nevertheless be formed. Remarkably, it was found that the compounds of formula (**2a**), (**2b**) and (**2c**) form stable salts with organic acids which is surprising as it is known from prior art that ketales are instable in the presence of acids. Moreover, the salts of the present invention are not only stable at room temperature but remain stable even during recrystallization from an organic solvent carried out at higher temperatures.

According to the present invention oxalic acid or pivalic acid may be used for salt formation. Further examples are the oxalic acid salt of compound (**2a**), the pivalic acid salt of compound (**2a**), the oxalic acid salt of compound (**2b**), the pivalic acid salt of compound (**2b**), the oxalic acid salt of compound (**2c**) and the pivalic acid salt of compound (**2c**). The acid salts of the 2-propyl esters of general formula (**2**) are isolated in high purity, i.e. from 90-99.9%, preferably from 95-99.9%, more preferably higher than 97%. Moreover the salts of the present invention are stable and may be stored for a long period of time without decomposition.

In a second aspect, the present invention relates to a method for the preparation of a salt of an organic acid with a compound of general formula (**2**) 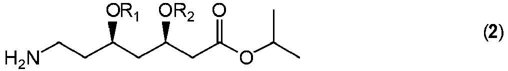 wherein R₁ and R₂ are independently chosen from the list consisting of ethyl, hydrogen, methyl and propyl or combined into a diol protecting group such that the compound of general formula (**2**) is represented as a compound of formula (**2a**), (**2b**) or (**2c**) 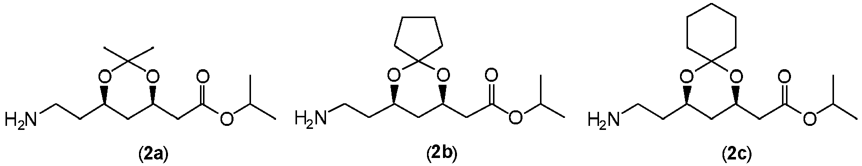 comprising reacting said compound of general formula (**2**) with an organic acid in an organic solvent, wherein said organic acid is oxalic acid or pivalic acid. The reaction may be carried out in an apolar, dipolar aprotic or protic solvent. As reaction medium an aliphatic hydrocarbon, aromatic hydrocarbon, halogenated hydrocarbon, ester, nitrile, alcohol or ether may be used. Examples are hexane, heptane, petroleumether, toluene, benzene, xylene, dichloromethane, chloroform, ethyl acetate, acetonitrile, methanol, ethanol, isopropanol, tetrahydrofurane, dioxane and diethyl ether. A solvent mixture may also be used as reaction medium; examples are heptane and toluene, hexane and toluene, hexane, toluene and tetrahydrofurane, heptane, toluene and tetrahydrofurane and hexane and diethyl ether. The compound of general formula (**2**) and the organic acid may be reacted in the form of solutions formed with the same solvent.

The compound of general formula (**2**) and the organic acid may be reacted in a molar ratio of 0.5-5, or 0.5-2 or 0.5-1.2. The compound of general formula (**2**) and the organic acid may be admixed at room temperature and the reaction may be performed under heating or at room temperature. The reaction may be carried out at the boiling point of the reaction mixture. Work up of the reaction mixture may be by cooling the reaction mixture, isolating the precipitated salt of the compound of the formula (**2**) by centrifugation, decantation, filtration and/or sedimentation, washing the salt with an organic solvent and drying. The salt may be purified by recrystallization. As starting material a crude compound of the general formula (**2**) may be is used in which case the laborious, expensive and complicated purification of the compound of general formula (**2**) is advantageously eliminated.

According to the present invention the 2-propyl esters of general formula (**2**) are purified by recrystallization as a salt which can be carried out significantly easier than alternate techniques such as fractionated distillation in high vacuo or column chromatography. The present invention provides a product of higher purity than the prior art methods. The method of the present invention is easily applicable on industrial scale as there is no requirement for dedicated equipment such as distillation- or chromatography equipment.

In a third aspect the high purity salts of the first aspect may be used in the preparation of atorvastatin meeting the requirements of international standards such as the Pharmacopoeia. The salts can be used as such in the conversion steps to atorvastatin. Also, the salts can be converted in a separate step to the highly pure amine and used as free amine in the subsequent steps to atorvastatin. Surprisingly, it has been found that application of the 2-propyl esters purified by salt formation give higher yields in the preparation of atorvastatin compared to situation wherein unpurified 2-propyl esters are applied.

### EXAMPLES

### Example 1

### Preparation of (4R, 6R)-1,3-dioxane-4-acetic acid, 6-(2-cyanomethyl)-2,2-dimethyl-1-methylethylester

A reactor was charged with water (160 g), NaCN (75.3 g, 1.48 mol) and NaOH (1.2 g, 0.03 mol). The reaction mixture was stirred for 30 min at 25°C to give a clear solution. (4*R*, 6*S*)-4-hydroxy-6-chloromethyl-tetrahydropyran-2-one was added (100 g, 0.59 mol, for preparation see WO 2002/06266) in 1 h at 25-30°C. Stirring was continued for 20 h at 30°C. The reaction mixture was cooled to 20°C and in 1 h, 37% aqueous HCl (104 g) was added keeping the temperature < 30°C (Note: the HCN generated at this step is scrubbed into a solution of aqueous sodium hypochlorite). Stirring was continued for 30 min. The reaction mixture was added in 30 min to 2-propanol (780 g) and stirred for 30 min. Then under vacuum (500-550 mbar, 40-45°C), 620 g of distillate was collected. Fresh 2-propanol (780 g) was added and again 620 g of distillate collected under vacuum (500-550 mbar, 40-45°C). After charging fresh 2-propanol (780 g), 20% HCl in 2-propanol (27 g) was added to the reaction mass and 620 g of distillate was collected under vacuum (500-550 mbar, 40-45°C). The reaction mixture was kept for 6 h at 50-55°C. Then cooled to 25-30°C and solid NaHCO₃ (100 g) was added. The reaction mass was further cooled to 15-20°C and dimethoxypropane (308 g, 2.9 mol) added. The reaction mixture was stirred for 1 h. The salts were filtered and washed with 2-propanol (78 g). The obtained solution was concentrated under vacuum. To the resulting brown oil was added methyl-*tert*-butyl ether (680 g), water (500 g) and NaHCO₃ (10 g). The methyl-*tert*-butyl ether was separated and washed with water (2 x 100 g). The methyl-*tert*-butyl ether phase containing the product was treated with 10 g active carbon for 30 min. After filtration of the carbon, the methyl-*tert*-butyl ether phase was concentrated under vacuum to give the product as yellow oil (111.9 g, GC assay 92%, yield 68%). This oil was used in the next step without further purification.

### Example 2

### Preparation of the oxalic acid salt of (4R, 6R)-1,3-dioxane-4-acetic acid, 6-(2-aminomethyl)-2,2-dimethyl-,1-methylethylester

(4*R*, 6*R*)-1,3-Dioxane-4-acetic acid, 6-(2-cyanomethyl)-2,2-dimethyl-,1-methylethylester (40 g based on 100% assay) was dissolved in 2-propanol (200 g) and 25% aqueous NH₃ added (32 mL). Raney Nickel (11 g, washed with 2-propanol, 2 x 50 g) was added. The reaction mixture was heated until 35°C and flushed 3 times with nitrogen and with hydrogen. The reaction mass was stirred under 12 bar hydrogen at 35°C for 7 h, then hydrogenated for another 15 h at 55°C. Raney Nickel is removed by filtration and washed with 2-propanol (80 g). The filtrate is concentrated to remove 2-propanol and water/NH₃. The residue was taken up in methanol (175 g) and neutralized with a 15% solution of oxalic acid in methanol until pH = 7. The reaction mixture is heated until a clear solution is obtained. After cooling to 25°C, 2-propanol (200 g) is added in 1 h. The resulting slurry is stirred for 6 h. The oxalic acid salt of (4*R*, 6*R*)-1,3-dioxane-4-acetic acid, 6-(2-aminomethyl)-2,2-dimethyl-,1-methylethylester is filtered and washed with 2-propanol (2 x 25 g). The product was obtained as a white solid (36.8 g, yield 77%).

### Example 3

### Preparation of 2-((4R, 6R)-6-(2-(3-(phenylcarbamoyl)-5-(4-fluorophenyl)-2-isopropyl-4-phenyl-1H-pyrrol-1-yl)ethyl)-2,2-dimethyl-1,3-dioxan-4-yl)acetic acid 1-methylethylester from the oxalic acid salt of (4R, 6R)-1,3-dioxane-4-acetic acid, 6-(2-aminomethyl)-2,2-dimethyl-,1-methylethylester and 2-[2-(4-fluorophenyl)-2-oxo-1-phenylethyl]-4-methyl-3-oxopentanoic acid phenylamide (DKT)

A reactor is charged with tetrahydrofuran (40 g), the oxalic acid salt of (4*R*, *6*R)-1,3-dioxane-4-acetic acid, 6-(2-aminomethyl)-2,2-dimethyl-, 1-methylethylester (6.3 g, 20.5 mmol amine) and the potassium salt of pivalic acid (3.5 g, 25.2 mmol). The reaction mixture was heated until 60°C and DKT (9.0 g, 21.7 mmol) was added followed by methyl-*tert*-butyl ether (40 g). The reaction mixture was heated to reflux under azeotropic water removal for 140 h. After cooling to 40-45°C, methyl-*tert*-butyl ether was added (200 g). The organic phase was cooled to 20-25°C and washed with 2.5% aqueous NaHCO₃ (2 x 100 g) and water (1 x 100 g). The methyl-*tert*-butyl ether solution was concentrated under vacuum to give an oily residue (~16 g). The residue was taken up in 2-propanol (30 g) and heated to 80°C to give a clear solution. Upon cooling to 55-60°C, the product precipitated. The slurry was further cooled in 1 h to 20-25°C under simultaneous addition of 2-propanol/water (40 g, 50/50 v/v). After stirring for 18 h, the product was isolated by filtration and washed with 2-propanol/water (3 x 7 g, 75/25 v/v). The wet-cake was added to 2-propanol (30 g), heated to reflux at 80°C until a clear solution was obtained. The solution was cooled in 2 h to 20-25°C, stirred for 3 h and the solids filtered and washed with 2-propanol (2 x 7 g). The product was dried under vacuum (8.8 g, 67% yield).

### Example 4

### Preparation of 2-((4R, 6R)-6-(2-(3-(phenylcarbamoyl)-5-(4-fluorophenyl)-2-isopropyl-4-phenyl-1H-pyrrol-1-yl)ethyl)-2,2-dimethyl-1,3-dioxan-4-yl)acetic acid 1-methylethylester from the oxalic acid salt of (4R, 6R)-1,3-dioxane-4-acetic acid, 6-(2-aminomethyl)-2,2-dimethyl-,1-methylethylester and 2-[2-(4-fluorophenyl)-2-oxo-1-phenylethyl]-4-methyl-3-oxopentanoic acid phenylamide (DKT)

A reactor was charged subsequently with water (10 g), the oxalic acid salt of (4*R*, *6*R)-1,3-dioxane-4-acetic acid, 6-(2-aminomethyl)-2,2-dimethyl-, 1-methylethylester (3.06 g, 10.1 mmol amine), DKT (4.17 g, 10.0 mmol), cyclohexane (50 g), toluene (40 g) and pivalic acid (580 mg, 5.7 mmol). The reaction mixture was heated until 70°C and solid NaHCO₃ (840 mg, 10 mmol) was added. The reaction mixture was heated to reflux under azeotropic water removal for 96 h. After cooling to 40-45°C, methyl-*tert*-butyl ether was added (100 g). The organic phase was cooled to 20-25°C and washed with 2.5% aqueous NaHCO₃ (2 x 50 g) and water (1 x 50 g). The methyl-*tert*-butyl ether solution was concentrated under vacuum to give an oily residue (~9 g). The residue was taken up in 2-propanol (25 g) and heated to 80°C to give a clear solution. Upon cooling to 55-60°C, the product precipitated. The slurry was further cooled in 1 h to 20-25°C under simultaneous addition of 2-propanol/water (20 g, 50/50 v/v). After stirring for 18 h, the product was isolated by filtration and washed with 2-propanol/water (3 x 5 g, 75/25 v/v). The white solid was dried under vacuum (3.4 g, 53% yield).

### Example 5

### Preparation of (4R, 6R)-1,3-dioxane-4-acetic acid, 6-(2-aminomethyl)-2,2-dimethyl-1-methylethylester from the oxalic acid salt of (4R, 6R)-1,3-dioxane-4-acetic acid, 6-(2-aminomethyl)-2,2-dimethyl-,1-methylethylester

The oxalic acid salt of (4*R*, 6*R*)-1,3-dioxane-4-acetic acid, 6-(2-aminomethyl)-2,2-dimethyl-,1-methylethylester (40 g, 0.13 mol amine) is added to water (125 g) at 20-25°C. To the stirred suspension is added a saturated aqueous Na₂CO₃ solution until pH = 10.9. The aqueous phase is extracted with methyl-*tert*-butyl ether (2 x 75 g).
The combined methyl-*tert*-butyl phases are concentrated under vacuum to give the amine as oil (32.3 g, 95% yield, assay 98%).

### Example 6

### Preparation of 2-((4R, 6R)-6-(2-(3-(phenylcarbamoyl)-5-(4-fluorophenyl)-2-isopropyl-4-phenyl-1H-pyrrol-1-yl)ethyl)-2,2-dimethyl-1,3-dioxan-4-yl)acetic acid 1-methylethylester

*(4R*, *6R)*-1,3-Dioxane-4-acetic acid, 6-(2-aminomethyl)-2,2-dimethyl-1-methylethylester (obtained from the oxalic acid salt as described in Example 5; 25.6 g, assay 98%, 98 mmol) and DKT (40.9 g, 98 mmol) were added to a stirred mixture of heptane (200 g) and tetrahydrofuran (140 g). Pivalic acid (6.6 g, 65 mmol) was added to the slurry and the mixture was heated to reflux under azeotropic water removal. About 100 g of a heptane/tetrahydrofuran/water mixture was distilled in 24 h, which was replaced by addition of fresh heptane (100 g). Azeotropic distillation was continued for 48 h. After cooling to 20-25°C, methyl-*tert*-butylether (200 g) was added. The organic phase containing the product was washed with 2.5% aqueous NaHCO₃ (150 mL) and 1 N aqueous HCl (150 mL). The organic phase was concentrated under vacuum. The residue was taken up in 2-propanol (350 g) and heated to 75-80°C to give a clear solution. Water (110 g) was added in 2 h, while allowing the reaction mixture to cool to 20-25°C. The resulting slurry was stirred for 4 h at 20-25°C.
The product was isolated by filtration and washed with 2-propanol (80/20 v/v, 3 x 50 g). After drying the product was obtained as a white solid (39.9 g, 64% yield).

### Example 7

### Preparation of atorvastatin calcium from 2-((4R, 6R)-6-(2-(3-(phenylcarbamoyl)-5-(4-fluorophenyl)-2-isopropyl-4-phenyl-1 H-pyrrol-1-yl)ethyl)-2,2-dimethyl-1,3-dioxan-4-yl)acetic acid 1-methylethylester

2-((4*R*,6*R*)-6-(2-(3-(Phenylcarbamoyl)-5-(4-fluorophenyl)-2-isopropyl-4-phenyl-1*H-*pyrrol-1-yl)ethyl)-2,2-dimethyl-1,3-dioxan-4-yl)acetic acid 1-methylethylester (12.0 g, 18.8 mmol, from Example 6) was added to methanol (200 g). The mixture was stirred at 37°C until a clear solution was obtained followed by cooling to 30°C. Next, 1 N aqueous HCl (30 mL) was added in 15 min and the resulting reaction mixture stirred for 3h at 25°C. To the mixture, 2.5 N aqueous NaOH (24 mL) was added in 15 h at 25-30°C, followed by heating to 38°C. After stirring for 1 h, the clear solution was concentrated to give 55 g of an oily residue. Water (150 g) and methanol (20 g) were added. The aqueous phase was extracted with methyl-*tert*-butyl ether (2 x 50 g) followed by extraction with a mixture of ethyl acetate/cyclohexane (42 g ethyl acetate and 37 g cyclohexane, 50/50 v/v)). Thereafter, the aqueous phase was treated with 1.0 g active carbon for 15 min. The carbon was removed over a 0.45 µm filter and washed with methanol/water 210 g, 50/50 v/v). The reaction mixture was heated until 48°C. Atorvastatin calcium polymorph I seed (0.6 g) was added, followed by addition of a solution of Ca-acetate.H₂O (1.8 g) in water (60 g) in 60 min. The mixture was heated to 58°C. After 1 h, the slurry was cooled to 35°C, kept at this temperature for 2 h and the product isolated by filtration.
The white solid was dried 50°C under vacuum (10.3 g, yield 90%, 99.3% pure).

### Example 8

### Preparation of (4R, 6R)-1,3-dioxane-4-acetic acid, 6-(2-aminomethyl)-2,2-dimethyl-1-methylethylester

(*4R*, *6R)*-1,3-Dioxane-4-acetic acid, 6-(2-cyanomethyl)-2,2-dimethyl-,1-methylethyl-ester (100 g, assay 92%, 0.36 mol) was dissolved in a mixture of 2-propanol (500 g) and water (35 g). Ammonia gas was passed into the reaction mixture until 5% ammonia content. Then, Raney Nickel (15 g on dry basis, washed with water (1 x 100 g) and 2-propanol (2 x 50 g)) was added. 2-Propanol (50 g) was used to rinse the catalyst into the reactor. The reaction mixture was heated to 30°C and flushed 3 times with nitrogen and with hydrogen. The reaction mass was stirred under 12 bar hydrogen pressure for 8 h at 30-35°C and then slowly heated to 50-55°C and hydrogenated for 16 at 50-55°C at 12 bar hydrogen. The reaction mixture is cooled to 30°C and the hydrogen vented. Raney Nickel was removed by filtration and washed with 2-propanol (1 x 100 g). Concentrating under vacuum at 35-40°C gave the product as oil (98.4 g, assay 85.7%, yield 91%). The amine was used in the next step without further purification

### Example 9

### Preparation of the pivalic acid salt of (4R, 6R)-1,3-dioxane-4-acetic acid, 6-(2-aminomethyl)-2,2-dimethyl-1-methylethylester

To (*4R*, *6R)*-1,3-dioxane-4-acetic acid, 6-(2-aminomethyl)-2,2-dimethyl-1-methylethylester (98.0 g, assay 85.7%, 0.33 mol) was added 2-propanol (200 g). The reaction was stirred for 15 min, followed by addition of a solution of pivalic acid (37.8 g, 0.37 mol) in 2-propanol (100 g). The reaction mixture was stirred for 30 min at 25-30°C and 2-propanol was removed by distillation under vacuum at 35-40°C. Hexane (200 g) was added and the reaction mass was stirred for 30 min. Hexane was stripped by distillation, followed by addition of fresh hexane (300 g). The slurry was cooled to 0-5°C and stirred for 1 h. The solids were isolated by filtration at 0-5°C and washed with cold hexane (100 g, 0-5°C). After drying under vacuum, a pale yellow solid was obtained (108.2 g, 97.3 % pure, yield 88.2%)

### Example 10

### Preparation of 2-((4R, 6R)-6-(2-(3-(phenylcarbamoyl)-5-(4-fluorophenyl)-2-isopropyl-4-phenyl-1H-pyrrol-1-yl)ethyl)-2,2-dimethyl-1,3-dioxan-4-yl)acetic acid 1-methylethyl ester from the pivalic acid salt of (4R, 6R)-1,3-dioxane-4-acetic acid, 6-(2-aminomethyl)-2,2-dimethyl-,1-methylethylester and 2-[2-(4-fluorophenyl)-2-oxo-1-phenylethyl]-4-methyl-3-oxopentanoic acid phenylamide (DKT)

A reactor is charged with cyclohexane (625 g), DKT (121 g, 0.29 mol), the pivalic acid salt of (4*R, 6R*)-1,3-dioxane-4-acetic acid, 6-(2-aminomethyl)-2,2-dimethyl-1-methylethylester (100 g, assay 97.3, 0.27 mol) and N-methyl-pyrrolidone (50 g). The reaction mixture was heated to reflux under azeotropic water removal for 30 h at 80-82°C. After cooling to 50-55°C, the solution was concentrated under vacuum, methyl-*tert*-butylether (625 g) added and stirred until a clear solution was obtained. The methyl-*tert*-butyl ether phase was washed with 10% aqueous NaHCO₃ (360 g). The phases were separated and the methyl-*tert*-butyl ether phase washed again with 10% aqueous NaHCO₃ (100 g). The combined aqueous phases were washed with methyl-*tert*-butylether (2 x 75 g). The combined methyl-*tert*-butylether phases containing the product were washed with water (3 x 200 g). After carbon treatment (10 g), the methyl-*tert*-butyl ether solution was concentrated under vacuum to give an oily residue (~200 g). The residue was taken up in 2-propanol (600 g) and heated to 65-70°C to give a clear solution. Upon cooling to 50-55°C, the product precipitated and the slurry was cooled in 1 h to 30°C. Water (400 g) was added in 1h and the slurry cooled to 0-2°C. After stirring for 4 h, the product was isolated by filtration and washed with 2-propanol/water (100 g, 60/40 v/v). The wet-cake was added to 2-propanol (400 g), heated to reflux at 80°C until a clear solution was obtained. The solution was cooled in 2 h to 0-2°C, the solids filtered and washed with 2-propanol (40 g). After drying under vacuum, the product was obtained as a white solid (105.0 g, 57% yield, assay 98%).

### Example 11

### Comparative example with (4R, 6R)-1,3-dioxane-4-acetic acid, 6-(2-aminomethyl)-2,2-dimethyl-, 1-methylethylester as free base; preparation of 2-((4R, 6R)-6-(2-(3-(phenylcarbamoyl)-5-(4-fluorophenyl)-2-isopropyl-4-phenyl-1H-pyrrol-1-yl)ethyl)-2,2-dimethyl-1,3-dioxan-4-yl)acetic acid 1-methylethylester

(4*R*, 6*R)*-1,3-Dioxane-4-acetic acid, 6-(2-aminomethyl)-2,2-dimethyl-1-methylethylester (obtained as described in Example 2 without oxalic acid formation; 25.6 g, assay 86%, 85.0 mmol,) and DKT (37.2 g, 89.2 mmol) were added to cyclohexane (200 g). Pivalic acid (6.6 g, 65 mmol) was added to the slurry and the mixture was heated to reflux under azeotropic water removal. Cyclohexane was then removed by distiilation under vacuum. Then methyl-*tert*-butylether (200 g) was added. The organic phase containing the product was washed with 2.5% aqueous NaHCO₃ (2 x 150 mL) and water (2 x 150 mL). The organic phase was concentrated under vacuum. The residue was taken up in 2-propanol (350 g) and heated to 75-80°C to give a clear solution. Water (110 g) was added in 2 h, while allowing the reaction mixture to cool to 20-25°C. The resulting slurry was stirred for 4 h at 20-25°C. The product was isolated by filtration and washed with 2-propanol (80/20 v/v, 3 x 50 g). After drying the product was obtained as a white solid (29.4 g, 54% yield, assay 96%).

### Example 12

### Preparation of atorvastatin calcium from 2-((4R, 6R)-6-(2-(3-(phenylcarbamoyl)-5-(4-fluorophenyl)-2-isopropyl-4-phenyl-1H-pyrrol-1-yl)ethyl)-2,2-dimethyl-1,3-dioxan-4-yl)acetic acid 1-methylethylester

2-((4*R*,6*R*)-6-(2-(3-(phenylcarbamoyl)-5-(4-fluorophenyl)-2-isopropyl-4-phenyl-1*H*-pyrrol-1-yl)ethyl)-2,2-dimethyl-1,3-dioxan-4-yl)acetic acid 1-methylethylester (30 g, 47 mmol) was added to methanol (350 g). The mixture was stirred at 33-35°C until a clear solution was obtained followed by cooling to 26-28°C. Then 2.2 N aqueous HCl (36 mL) was added in 15 min and the resulting reaction mixture stirred for 2 h at 26-28°C. To the mixture, 0.6 N aqueous NaOH (207 mL) was added in 1 h keeping the temperature below 30°C. After stirring for 2h, the clear solution was concentrated under vacuum at 27-29°C until a slurry was obtained. Then water (300 g) and methyl-*t*-butyl ether (130 g) were added. The phases were separated. Next, the aqueous layer was extracted with a mixture of ethyl acetate/cyclohexane (215 g ethyl acetate and 185 g cyclohexane, 50/50 v/v). The phases were separated. Thereafter, the aqueous phase was treated with 3.0 g active carbon. The reaction mixture was heated until 45-50°C and H₂O (60 g) added. 3.0 g of atorvastatin calcium polymorph I seed was added, followed by addition in 1 h of a solution of 6.0 g Ca-acetate in water (150 g). The mixture was heated to 55-58°C and maintained at this temperature for 30 minutes. The slurry was cooled to 40-45°C and stirred for 3 h. The solid was isolated by filtration and re-slurried in water (400 g). The slurry was heated to 40°C, stirred for 1 h and filtered.
The white solid was dried at 50-55°C (24.1 g, yield 85 %).

## Claims

1. Salt of an organic acid with a compound of general formula (**2**) 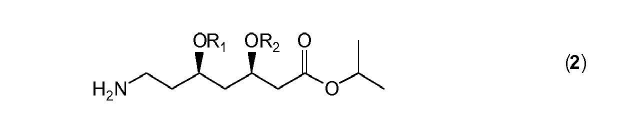 wherein R₁ and R₂ are independently chosen from the list consisting of ethyl, hydrogen, methyl and propyl or combined into a diol protecting group such that the compound of general formula (**2**) is represented as a compound of formula (**2a**), (**2b**) or (**2c**) 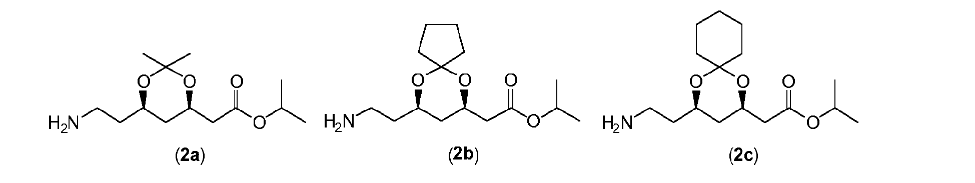 wherein said organic acid is oxalic acid or pivalic acid.

2. Salt according to claim 1 wherein said compound of general formula (**2**) is the compound of formula (**2a**).

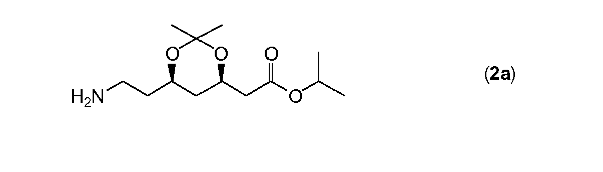

3. Method for the preparation of a salt of an organic acid with a compound of general formula (**2**) 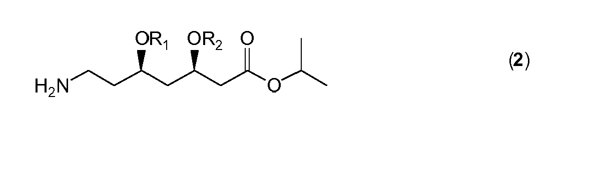 wherein R₁ and R₂ are independently chosen from the list consisting of ethyl, hydrogen, methyl and propyl or combined into a diol protecting group such that the compound of general formula (**2**) is represented as a compound of formula (**2a**), (**2b**) or (**2c**) 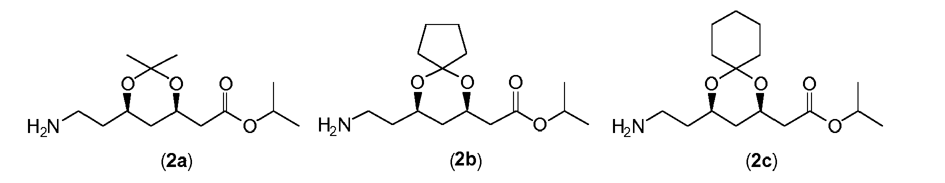 comprising reacting said compound of general formula (**2**) with an organic acid in an organic solvent, wherein said organic acid is oxalic acid or pivalic acid.

4. Use of a salt of an oxalic acid or pivalic acid with a compound of general formula (**2**) 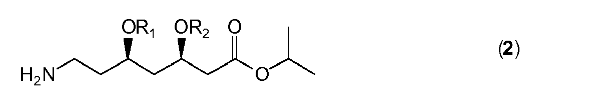 wherein R₁ and R₂ are independently chosen from the list consisting of ethyl, hydrogen, methyl and propyl or combined into a diol protecting group such that the compound of general formula (**2**) is represented as a compound of formula (**2a**), (**2b**) or (**2c**) 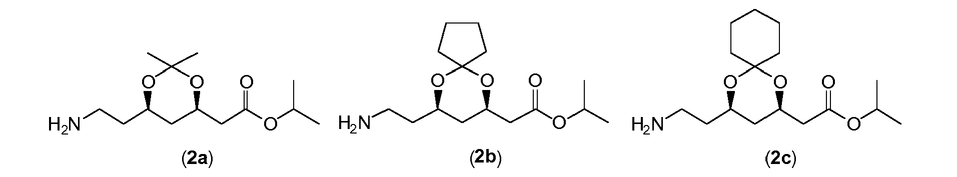 in the preparation of a compound of formula (**3**) 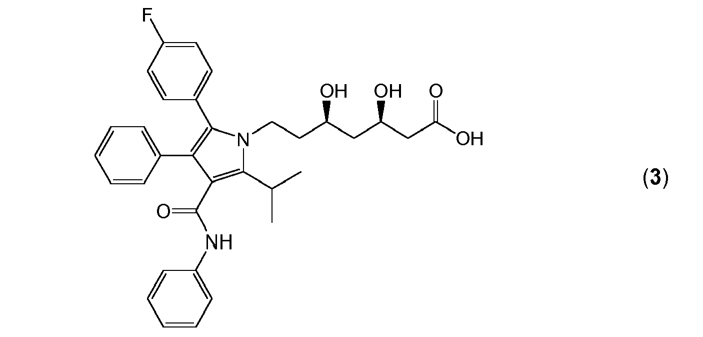 or a pharmaceutically acceptable salt or ester thereof.

5. Use according to claim 4 wherein said salt of an organic acid with a compound of general formula (**2**) is converted to the amine of general formula (**2**) prior to conversion into said compound of formula (**3**).

## Patentansprüche

1. Salz einer organischen Säure mit einer Verbindung der allgemeinen Formel (2) 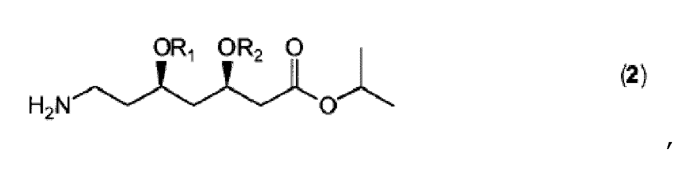 worin R₁ und R₂ unabhängig aus der Reihe Ethyl, Wasserstoff, Methyl und Propyl ausgewählt sind oder zu einer Diolschutzgruppe kombiniert sind, so dass die Verbindung der allgemeinen Formel (**2**) einer Verbindung der Formel (**2a**), (**2b**) oder (**2c**) 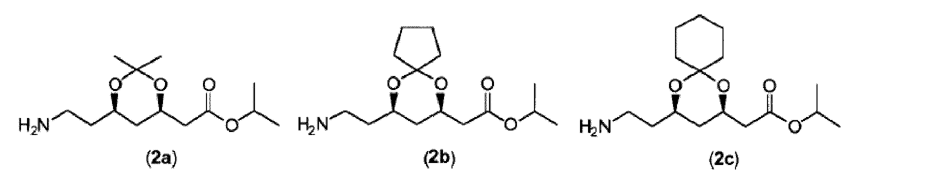 entspricht, wobei es sich bei der organischen Säure um Oxalsäure oder Pivalinsäure handelt.

2. Salz nach Anspruch 1, wobei es sich bei der Verbindung der allgemeinen Formel (**2**) um die Verbindung der Formel (**2a**) handelt.

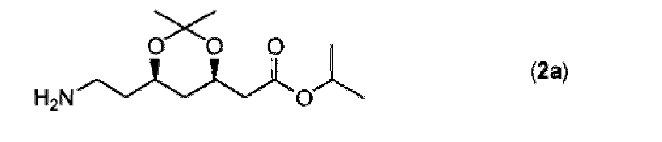

3. Verfahren zur Herstellung eines Salzes einer organischen Säure mit einer Verbindung der allgemeinen Formel (2) 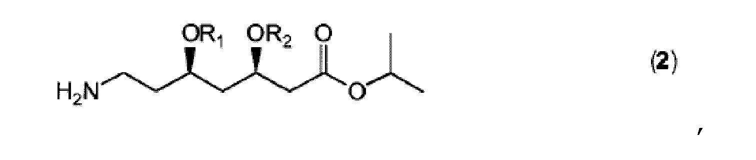 worin R₁ und R₂ unabhängig aus der Reihe Ethyl, Wasserstoff, Methyl und Propyl ausgewählt sind oder zu einer Diolschutzgruppe kombiniert sind, so dass die Verbindung der allgemeinen Formel (**2**) einer Verbindung der Formel (**2a**), (**2b**) oder (**2c**) 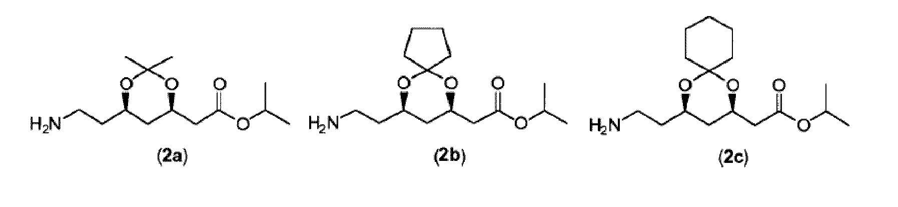 entspricht,
bei dem man die Verbindung der allgemeinen Formel (**2**) mit einer organischen Säure in einem organischen Lösungsmittel umsetzt, wobei es sich bei der organischen Säure um Oxalsäure oder Pivalinsäure handelt.

4. Verwendung eines Salzes der Oxalsäure oder Pivalinsäure mit einer Verbindung der allgemeinen Formel (**2**) 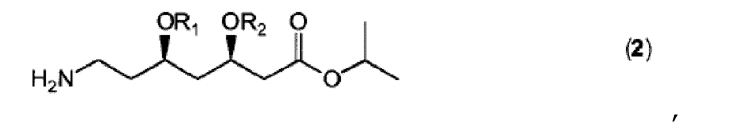 worin R₁ und R₂ unabhängig aus der Reihe Ethyl, Wasserstoff, Methyl und Propyl ausgewählt sind oder zu einer Diolschutzgruppe kombiniert sind, so dass die Verbindung der allgemeinen Formel (**2**) einer Verbindung der Formel (**2a**), (**2b**) oder (**2c**) 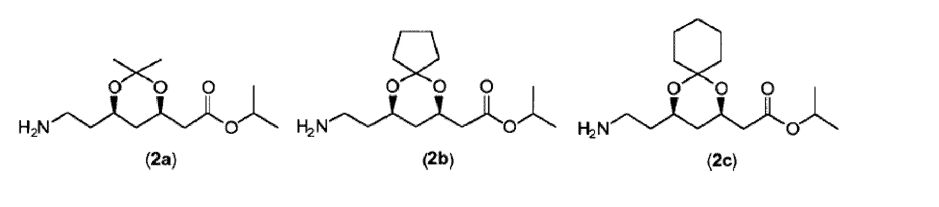 entspricht,
in der Herstellung einer Verbindung der Formel (**3**) 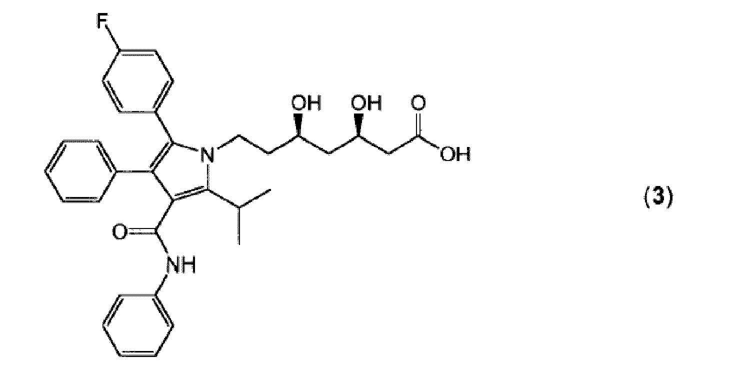 oder eines pharmazeutisch unbedenklichen Salzes oder Esters davon.

5. Verwendung nach Anspruch 4, wobei das Salz einer organischen Säure mit einer Verbindung der allgemeinen Formel (**2**) vor der Umwandlung in die Verbindung der Formel (**3**) in das Amin der allgemeinen Formel (**2**) umgewandelt wird.

## Revendications

1. Sel d'un acide organique avec un composé de formule générale (2) 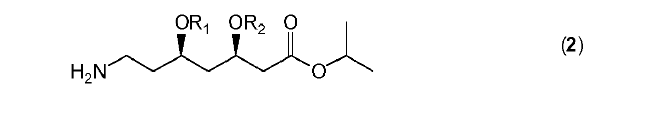 dans laquelle R₁ et R₂ sont choisis indépendamment dans la liste constituée par éthyle, hydrogène, méthyle et propyle ou combinés en un groupement protecteur de diol, de sorte que le composé de formule générale (2) soit représenté sous forme de composé de formule (2a), (2b) ou (2c) 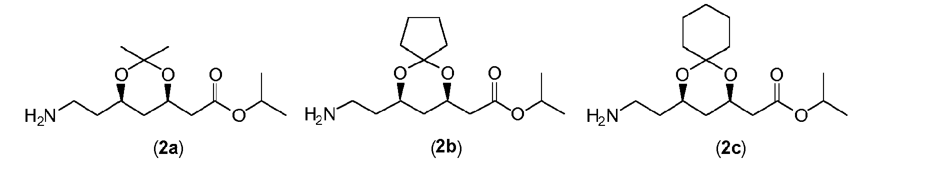 où ledit acide organique est l'acide oxalique ou l'acide pivalique.

2. Sel selon la revendication 1, **caractérisé en ce que** ledit composé de formule générale (2) est le composé de formule générale (2a)

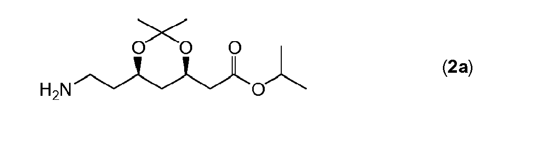

3. Méthode de préparation d'un sel d'un acide organique avec un composé de formule générale (2) 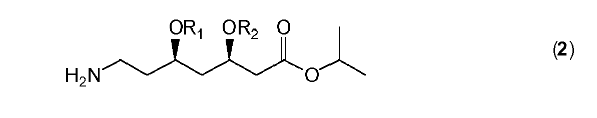 dans laquelle R₁ et R₂ sont choisis indépendamment dans la liste constituée par éthyle, hydrogène, méthyle et propyle ou combinés en un groupement protecteur de diol, de sorte que le composé de formule générale (2) soit représenté sous forme de composé de formule (2a), (2b) ou (2c) 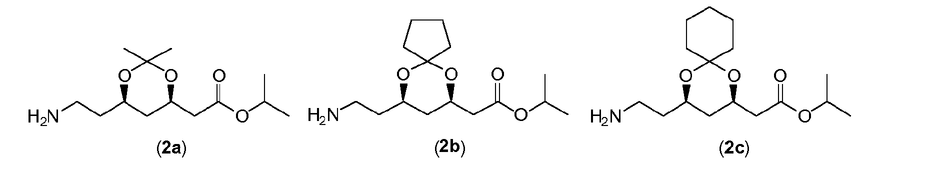 comprenant la réaction dudit composé de formule générale (2) avec un acide organique dans un solvant organique, où ledit acide organique est l'acide oxalique ou l'acide pivalique.

4. Utilisation d'un sel d'acide oxalique ou d'acide pivalique avec un composé de formule générale (2) 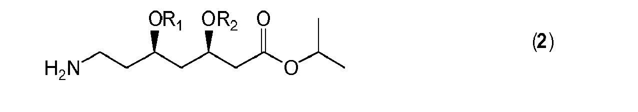 dans laquelle R₁ et R₂ sont choisis indépendamment dans la liste constituée par éthyle, hydrogène, méthyle et propyle ou combinés en un groupement protecteur de diol, de sorte que le composé de formule générale (2) soit représenté sous forme de composé de formule (2a), (2b) ou (2c) 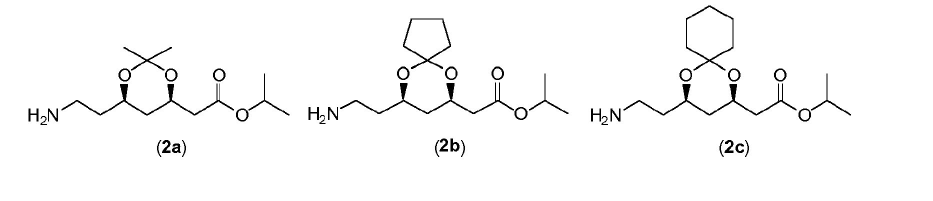 dans la préparation d'un composé de formule (3) 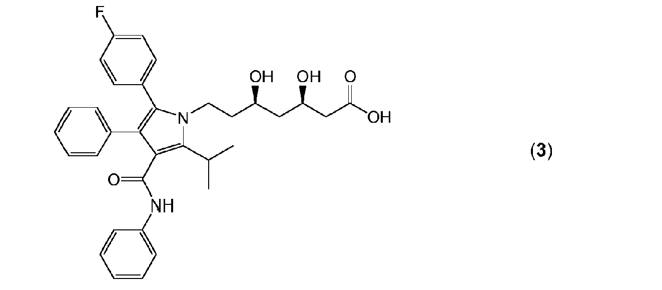 ou un sel ou ester pharmaceutiquement acceptable de celui-ci.

5. Utilisation selon la revendication 4, **caractérisée en ce que** ledit sel d'un acide organique avec un composé de formule générale (2) est converti en amine de formule générale (2) avant conversion en ledit composé de formule (3).
